# EUROPEAN PATENT APPLICATION

(11) **EP 2 684 964 A1**
(43) Date of publication of application: **15.01.2014**
(21) Application number: 12005067.9
(22) Date of filing: 09.07.2012
(51) Int. Cl.: C12Q 1/68

(54) **Microarray-based methods for identifying single nucleotides at specific positions in nucleic acids**

(71) Applicant: OakLabs GmbH, 16761 Hennigsdorf (DE)
(72) Inventor: Schad, Martina, 10117 Berlin (DE); Kallarackal, Jim, 10117 Berlin (DE)
(74) Representative: Ricker, Mathias

(57) **Abstract**

The present invention relates to methods for identifying single nucleotides at specific positions in at least one target nucleic acid. The methods involve the synthesis of a plurality of different primers using a first DNA microarray, the template-dependent extension of the primers by a single detectably labeled nucleotide, the hybridization of the labeled and extended primers to oligonucleotides on a second DNA microarray, and the determination of the nucleotide to be identified by detecting the detectable label. The methods of the present invention are suitable for determining the genotype of an organism at a single nucleotide polymorphic site, assessing the genetic similarity between organisms or populations of organisms, identifying at least one single nucleotide polymorphic site as a diagnostic disease marker, and diagnosing a disease in a subject.

## Description

### FIELD OF THE INVENTION

The present invention relates to methods for identifying single nucleotides at specific positions in at least one target nucleic acid. The methods involve the synthesis of a plurality of different primers using a first DNA microarray, the template-dependent extension of the primers by a single detectably labeled nucleotide, the hybridization of the labeled and extended primers to oligonucleotides on a second DNA microarray, and the determination of the nucleotide to be identified by detecting the detectable label. The methods of the present invention are suitable for determining the genotype of an organism at a single nucleotide polymorphic site, predicting a monogenic or complex trait of a human, animal or plant, assessing the genetic similarity between organisms or populations of organisms, identifying at least one single nucleotide polymorphic site as a diagnostic disease marker, and diagnosing a disease in a subject.

### BACKGROUND OF THE INVENTION

Although many techniques are known that allow for the analysis of single nucleotide polymorphisms (SNPs), these techniques still suffer from various drawbacks, such as, for example they may be complex, difficult to handle, time-consuming, costly, and lack the desired sensitivity, specificity and/nr selectivity. Among the various analytical methods used to detect SNPs, hybridization-based techniques have been developed which have obviated some of the mentioned drawbacks but have not completely solved all problems.

Some hybridization-based techniques that have been developed are based on a template-dependent, primer extension reaction. These techniques include methods employing specific primers which span the region to be queried for the single nucleotide mutation (see, e.g., US 5,578,458 and US 4,851,331) and techniques using primers that hybridize immediately adjacent and upstream of the position to be queried for the single nucleotide mutation (see, e.g., US 5,888,819 and US 6,004,744).

The latter technique is commonly referred to as single base extension (SBE) method and exploits the high fidelity of the polymerase that is used in the template-dependent extension of a specific SBE primer by a single nucleotide. The incorporated single nucleotide is a chain-terminating nucleotide that is detectably labeled. The detection of the label enables the identification of a single nucleotide of interest, in particular a SNP, at a specific position along a nucleic acid.

More recently, microarrays have become available which carry a number of different biomolecules arranged in a regular pattern. These microarrays enable the assessment of many different parameters in a high-throughput fashion. For nucleic acid analysis, DNA microarrays have been developed. DNA microarrays comprise small supports (e.g., glass slides or silicon chips) onto which thousands of oligonucleotides are immobilized at fixed locations in a regular pattern (arrays). These DNA microarrays work by exploiting the ability of the oligonucleotides to specifically hybridize to the nucleic acid templates from which they originate.

At present, there are a number of technologies commercially available for the analysis of SNPs, such as the GoldenGate^{®} Genotyping Assay (Illumina), the iSelect^{®} Genotyping Assay (lllumina), the PyroMark PCR Kit (Qiagen), the SNPtype^{™} Assay (Fluidigm), the MassARRAY^{®} SNP Genotyping Assay (Sequenom), the OpenArray^{®} technology (Applied Biosystems), etc., which can be used for analyzing a high number of DNA markers and/or a high number of samples. However, despite these achievements, the currently available methods for identifying SNPs are still rather expensive, especially when it is desired to analyse a high numbers of SNPs for a relatively low number of nucleic acid samples. Hence, there is still room for improvements with respect to cost-efficiency of large-scale screening methods without impairing the required high specificity and high sensitivity.

Accordingly, there remains a need in the art for a cost-efficient and reliable method for identifying single nucleotides, in particular SNPs, at specific positions of nucleic acids.

### SUMMARY OF THE INVENTION

The present invention relates to methods for producing single base extension (SBE) primers and to methods for identifying single nucleotides at specific positions in at least one target nucleic acid by a single base extension (SBE) reaction. The single nucleotide to be identified is typically a SNP. The methods of the present invention are very cost-efficient while at the same time being highly reliable. Therefore, the methods offer the potential of a variety of applications, such as identifying and analyzing SNPs to detect diseases associated with SNPs or predicting monogenic or complex traits of humans, animals or plants.

More specifically, the present invention relates to a method for producing single base extension (SBE) primers, the method comprising the steps of:
(a) providing a first DNA microarray, the first DNA microarray having a plurality of different immobilized DNA oligonucleotides assigned to different respective microarray locations, each of the immobilized DNA oligonucleotides comprising a specific DNA sequence present in the target nucleic acid immediately upstream of the nucleotide to be identified;
(b) enzymatically synthesizing, on the first DNA microarray, DNA strands complementary to the immobilized DNA oligonucleotides;
(c) separating the complementary DNA strands synthesized in step (b) from the first DNA microarray;
(d) amplifying the separated complementary DNA strands by polymerase chain reaction (PCR) to produce a PCR product;
(e) cleaving the PCR product immediately adjacent to, and downstream of, said specific DNA sequence to obtain cleaved DNA strands having a 3' end terminating one nucleotide before the nucleotide to be identified, the cleaved DNA strands being suited for use as single base extension (SBE) primers.

Furthermore, there is provided a method for identifying single nucleotides at specific positions in at least one target nucleic acid, the method comprising the steps of:
(a) providing a first DNA microarray, the first DNA microarray having a plurality of different immobilized DNA oligonucleotides assigned to different respective microarray locations, each of the immobilized DNA oligonucleotides comprising a specific DNA sequence present in the target nucleic acid immediately upstream of the nucleotide to be identified;
(b) enzymatically synthesizing, on the first DNA microarray, DNA strands complementary to the immobilized DNA oligonucleotides;
(c) separating the complementary DNA strands synthesized in step (b) from the first DNA microarray;
(d) amplifying the separated complementary DNA strands by polymerase chain reaction (PCR) to produce a PCR product;
(e) cleaving the PCR product immediately adjacent to, and downstream of, said specific DNA sequence to obtain cleaved DNA strands having a 3' end terminating one nucleotide before the nucleotide to be identified, the cleaved DNA strands being suited for use as single base extension (SBE) primers;
(f) extending the SBE primers of step (e) by a single nucleotide by means of hybridizing the SBE primers to said at least one target nucleic acid and incubating the formed hybridization complexes of target nucleic acid and SBE primer in the presence of at least one detectably labeled chain-terminating nucleotide and a polymerase to cause the incorporation of the detectably labeled chain-terminating nucleotide at the 3' terminus of the SBE primers;
(g) providing a second DNA microarray, the second DNA microarray having a plurality of different immobilized DNA antisense oligonucleotides assigned to different respective microarray locations, each of the assigned DNA antisense oligonucleotides being complementary to at least a portion of said different immobilized DNA oligonucleotides of the first DNA microarray;
(h) hybridizing the extended SBE primers to the different immobilized DNA antisense oligonucleotides;
(i) identifying the incorporated chain-terminating nucleotides at the 3' terminus of the extended SBE primers by detecting the detectable label of said chain-terminating nucleotides for each of the extended SBE primers hybridized to the different immobilized and assigned DNA antisense oligonucleotides of the second microarray, thereby determining the nucleotide to be identified.

The present invention further relates to an alternative method for producing single base extension (SBE) primers, the method comprising the steps of:
(a) providing a first DNA microarray, the first DNA microarray having a plurality of different immobilized DNA oligonucleotides assigned to different respective microarray locations, each of the assigned DNA oligonucleotides comprising a specific DNA sequence present in the target nucleic acid immediately upstream of the nucleotide to be identified;
(b') separating the immobilized assigned DNA oligonucleotides from the first DNA microarray;
(c') enzymatically synthesizing, in a reaction vessel, DNA strands complementary to the separated DNA oligonucleotides;
(d') amplifying the synthesized complementary DNA strands by polymerase chain reaction (PCR) to produce a PCR product;
(e) cleaving the PCR product immediately adjacent to, and downstream of, said specific DNA sequence to obtain cleaved DNA strands having a 3' end terminating one nucleotide before the nucleotide to be identified, the cleaved DNA strands being suited for use as single base extension (SBE) primers.

Further disclosed herein is an alternative method for identifying single nucleotides at specific positions in at least one target nucleic acid, the method comprising the steps of:
(a) providing a first DNA microarray, the first DNA microarray having a plurality of different immobilized DNA oligonucleotides assigned to different respective microarray locations, each of the assigned DNA oligonucleotides comprising a specific DNA sequence present in the target nucleic acid immediately upstream of the nucleotide to be identified;
(b') separating the immobilized assigned DNA oligonucleotides from the first DNA microarray;
(c') enzymatically synthesizing, in a reaction vessel, DNA strands complementary to the separated DNA oligonucleotides;
(d') amplifying the synthesized complementary DNA strands by polymerase chain reaction (PCR) to produce a PCR product;
(e) cleaving the PCR product immediately adjacent to, and downstream of, said specific DNA sequence to obtain cleaved DNA strands having a 3' end terminating one nucleotide before the nucleotide to be identified, the cleaved DNA strands being suited for use as single base extension (SBE) primers;
(f) extending the SBE primers of step (e) by a single nucleotide by means of hybridizing the SBE primers to said at least one target nucleic acid and incubating the formed hybridization complexes of target nucleic acid and SBE primer in the presence of at least one detectably labeled chain-terminating nucleotide and a polymerase to cause the incorporation of the detectably labeled chain-terminating nucleotide at the 3' terminus of the SBE primers;
(g) providing a second DNA microarray, the second DNA microarray having a plurality of different immobilized DNA antisense oligonucleotides assigned to different respective microarray locations, each of the assigned DNA antisense oligonucleotides being complementary to at least a portion of said different immobilized DNA oligonucleotides of the first DNA microarray;
(h) hybridizing the extended SBE primers to the different immobilized DNA antisense oligonucleotides;
(i) determining the incorporated chain-terminating nucleotides at the 3' terminus of the extended SBE primers by detecting the detectable label of said chain-terminating nucleotides for each of the extended SBE primers hybridized to the different immobilized and assigned DNA antisense oligonucleotides of the second microarray, thereby determining the nucleotide to be identified.

Yet further, the present invention relates to an another alternative method for producing single base extension (SBE) primers, the method comprising the steps of:
(a) providing a first DNA microarray, the first DNA microarray having a plurality of different immobilized DNA oligonucleotides assigned to different respective microarray locations, each of the assigned DNA oligonucleotides comprising a specific DNA sequence present in the target nucleic acid immediately upstream of the nucleotide to be identified;
(b") enzymatically synthesizing, on the first DNA microarray, DNA strands complementary to the immobilized DNA oligonucleotides to form an immobilized, double-stranded DNA fragment;
(c1") cleaving off the double-stranded DNA fragment from the first DNA microarray;
(c2") ligating one linker or two linkers, one at each end, to the cleaved off double-stranded DNA fragment to obtain a ligated double-stranded DNA fragment;
(d") amplifying the ligated double-stranded DNA fragment by polymerase chain reaction (PCR) to produce a PCR product including a strand having the complement of a first universal sequence at one end and a second universal sequence at the other end;
(e) cleaving the PCR product immediately adjacent to, and downstream of, said specific DNA sequence to obtain cleaved DNA strands having a 3' end terminating one nucleotide before the nucleotide to be identified, the cleaved DNA strands being suited for use as single base extension (SBE) primers.

There is also disclosed another alternative method for identifying single nucleotides at specific positions in at least one target nucleic acid, the method comprising the steps of:
(a) providing a first DNA microarray, the first DNA microarray having a plurality of different immobilized DNA oligonucleotides assigned to different respective microarray locations, each of the assigned DNA oligonucleotides comprising a specific DNA sequence present in the target nucleic acid immediately upstream of the nucleotide to be identified;
(b") enzymatically synthesizing, on the first DNA microarray, DNA strands complementary to the immobilized DNA oligonucleotides to form an immobilized, double-stranded DNA fragment;
(c1") cleaving off the double-stranded DNA fragment from the first DNA microarray;
(c2") ligating one linker or two linkers, one at each end, to the cleaved off double-stranded DNA fragment to obtain a ligated double-stranded DNA fragment;
(d") amplifying the ligated double-stranded DNA fragment by polymerase chain reaction (PCR) to produce a PCR product including a strand having the complement of a first universal sequence attached to one end and a second universal sequence attached to the other end;
(e) cleaving the PCR product immediately adjacent to, and downstream of, said specific DNA sequence to obtain cleaved DNA strands having a 3' end terminating one nucleotide before the nucleotide to be identified, the cleaved DNA strands being suited for use as single base extension (SBE) primers;
(f) extending the SBE primers of step (e) by a single nucleotide by means of hybridizing the SBE primers to said at least one target nucleic acid and incubating the formed hybridization complexes of target nucleic acid and SBE primer in the presence of at least one detectably labeled chain-terminating nucleotide and a polymerase to cause the incorporation of the detectably labeled chain-terminating nucleotide at the 3' terminus of the SBE primers;
(g) providing a second DNA microarray, the second DNA microarray having a plurality of different immobilized DNA antisense oligonucleotides assigned to different respective microarray locations, each of the assigned DNA antisense oligonucleotides being complementary to at least a portion of said different immobilized DNA oligonucleotides of the first DNA microarray;
(h) hybridizing the extended SBE primers to the different immobilized DNA antisense oligonucleotides;
(i) determining the incorporated chain-terminating nucleotides at the 3' terminus of the extended SBE primers by detecting the detectable label of said chain-terminating nucleotides for each of the extended SBE primers hybridized to the different immobilized and assigned DNA antisense oligonucleotides of the second microarray, thereby determining the nucleotide to be identified.

In addition, the present invention relates to the use of the methods described above for determining the genotype of an organism at a single nucleotide polymorphic site, predicting a monogenic or complex trait of a human, animal or plant, a method for assessing the genetic similarity between a target organism or a target population of organisms and a reference organism and/or a reference population of organisms, a method for identifying at least one single nucleotide polymorphic site as a diagnostic disease marker, and a method for diagnosing a disease in a subject.

Furthermore, the present invention relates to a kit for identifying single nucleotides at specific positions in at least one target nucleic acid, comprising a first DNA microarray and a second DNA microarray as used in the methods of the present invention.

The present invention may be more fully understood by reference to the following detailed description of the present invention, the examples, and the accompanying drawings.

### BRIEF DESCRIPTION OF THE DRAWINGS

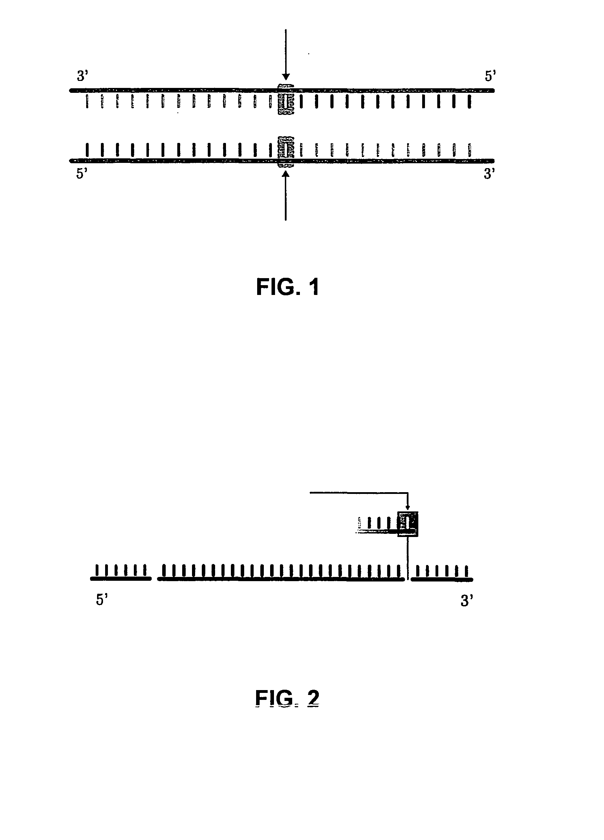

**FIG. 1** is a schematic illustration showing the base to be interrogated (i.e., a SNP) within the dashed, shaded box. The oligonucleotide design for the first DNA microarray preferably incorporates (a) the specific DNA sequence immediately adjacent to, and upstream of, the nucleotide to be interrogated of one of the two DNA strands (e.g., indicated by the lower arrow), and (b) the specific DNA sequence immediately adjacent to, and upstream of, the nucleotide complementary to the nucleotide to be interrogated of the other DNA strand (e.g., indicated by the upper arrow). The solid black vertical lines denote the specific DNA sequences immobilized on the first microarray.

**FIG. 2** is a schematic illustration showing the sequence of an exemplary oligonucleotide immobilized on the first microarray, comprising (a) one of the specific DNA sequences shown in FIG. 1, (b) the complement of a first universal sequence attached at the 3' side of the specific DNA sequence, and (c) a second universal sequence attached at the 5' side of the specific DNA sequence (the complement of the first universal sequence and the second universal sequence are set apart from the specific sequence by a small gap for reasons of illustration - they are, however, covalently linked). The nucleotide to be interrogated is not included in the oligonucleotide. The upper part of the illustration shows the notional position of the (absent) nucleotide to be interrogated as a white solid vertical dash within the shaded box.

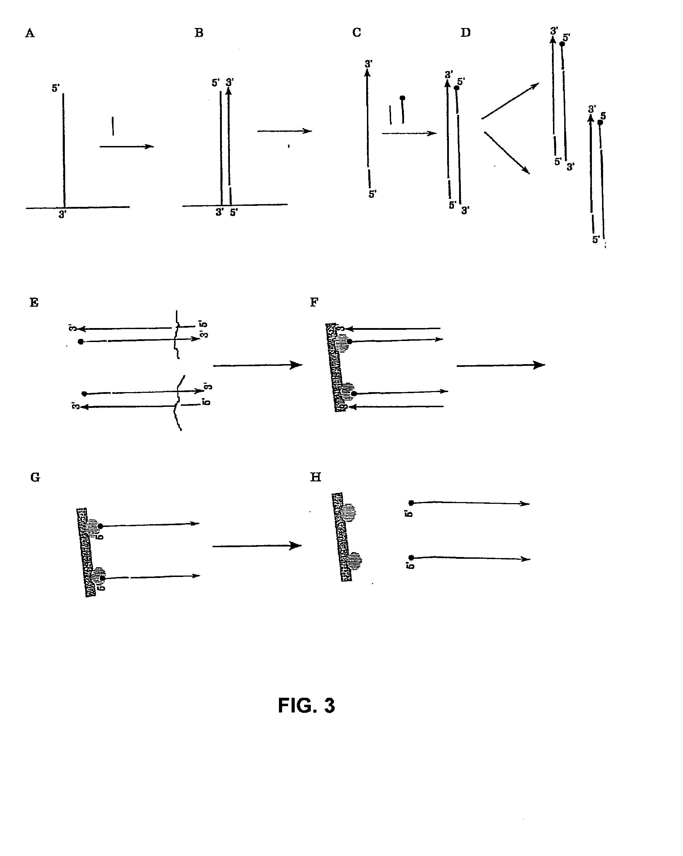

**FIG. 3** is a schematic overview of the synthesis of single base extension (SBE) primers according to an exemplary embodiment of the present invention. A and B: synthesis of DNA strands complementary to the oligonucleotides immobilized by their 3' ends on the first DNA microarray (synthesis array) using the complement of a first universal sequence as primer; C: isolation of synthesized DNA strands; D: PCR amplification using the first universal sequence and a desthiobiotinylated second universal sequence as PCR primers, resulting in the incorporation of desthiobiotin; E: restriction digestion for removal of first universal sequence and complement thereof; F: binding to streptavidin-coated magnetic beads; G: removal of non-desthiobiotinylated strands; H: recovery of the desthiobiotinylated strands for use as SBE primers in single-base primer extension. The following symbols are used: short straight line = first universal sequence; short straight line with a filled black circle at one end = second universal sequence modified with desthiobiotin at the 5' terminus; light grey ellipses on a dark grey bar = streptavidin-coated magnetic beads; irregular line = restriction site.

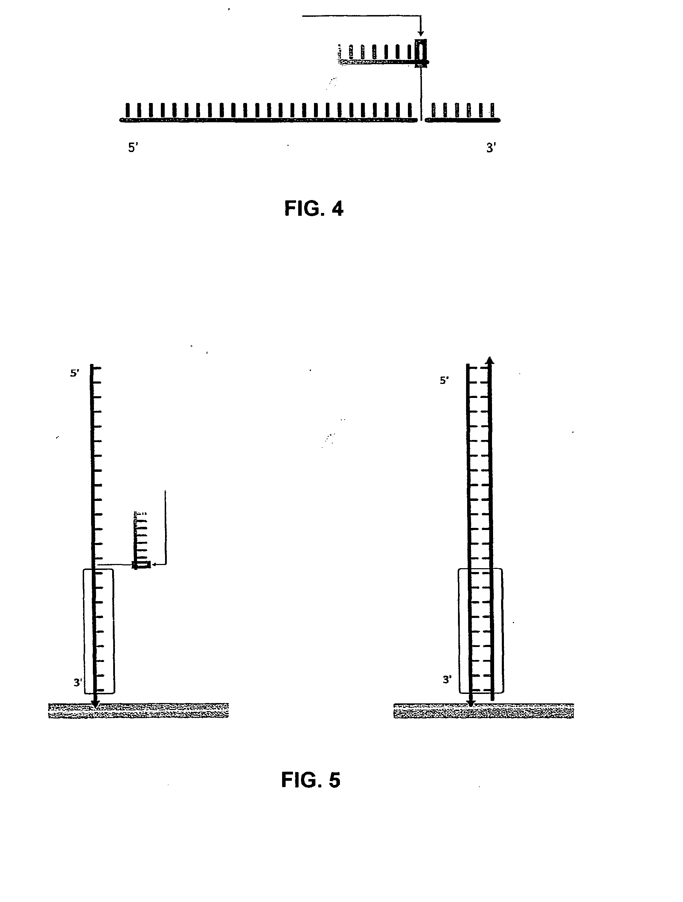

**FIG. 4** is a schematic illustration showing the sequence of another exemplary oligonucleotide immobilized on the first microarray, comprising (a) one of the specific DNA sequences shown in FIG. 1 and (b) the complement of a universal sequence at the 3' end of the specific DNA sequence. The nucleotide to be interrogated is not included in the oligonucleotide. The upper part of the illustration shows the notional position of the (absent) nucleotide to be interrogated as a white solid vertical dash within the shaded box.

**FIG. 5** is a schematic illustration showing the DNA microarray-based synthesis of complementary strands. The left part of the illustration shows an oligonucleotide immobilized with the 3' end on the support (e.g., a glas slide). The rectangle denotes the universal sequence. The arrow points to the notional position of the (absent) nucleotide to be interrogated depicted as a white solid vertical dash within the shaded box. On the right side of FIG. 5, there is shown the synthesized complementary strand in form of a double-stranded structure.

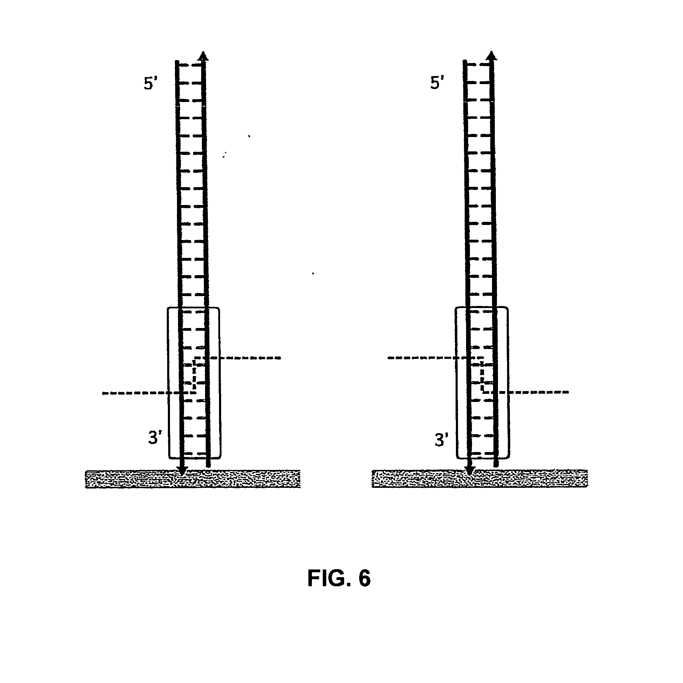

**FIG. 6** is a schematic illustration showing the cleavage of the double-stranded DNA fragment from the first DNA microarray by means of a restriction enzyme. As indicated, the restriction enzyme is preferably chosen such that the separated DNA fragment has either a 3' overhang (on the left) or a 5' overhang (on the right).

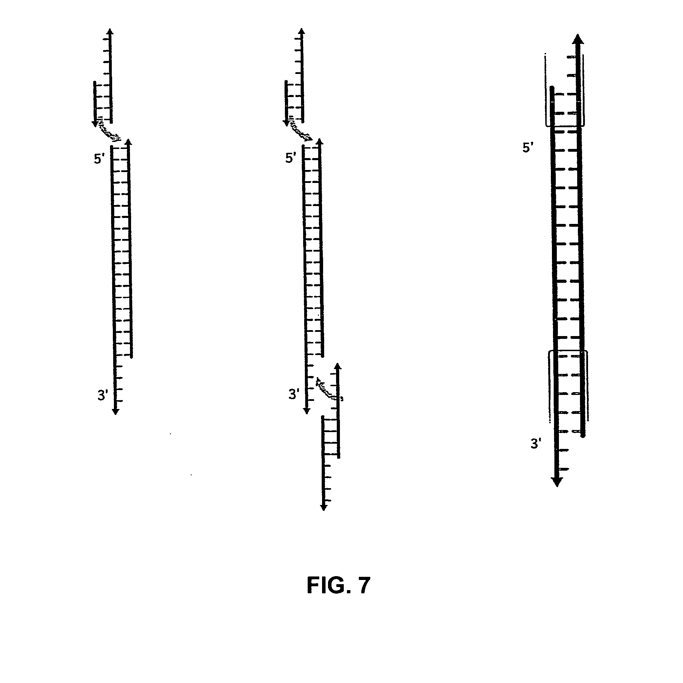

**FIG. 7** is a schematic illustration showing the ligation of one linker (see illustration on the left) or two linkers (see illustration in the middle). In addition, a final, ligated DNA fragment having a specific sequence flanked by universal but distinct sequence blocks on both ends is shown on the right.

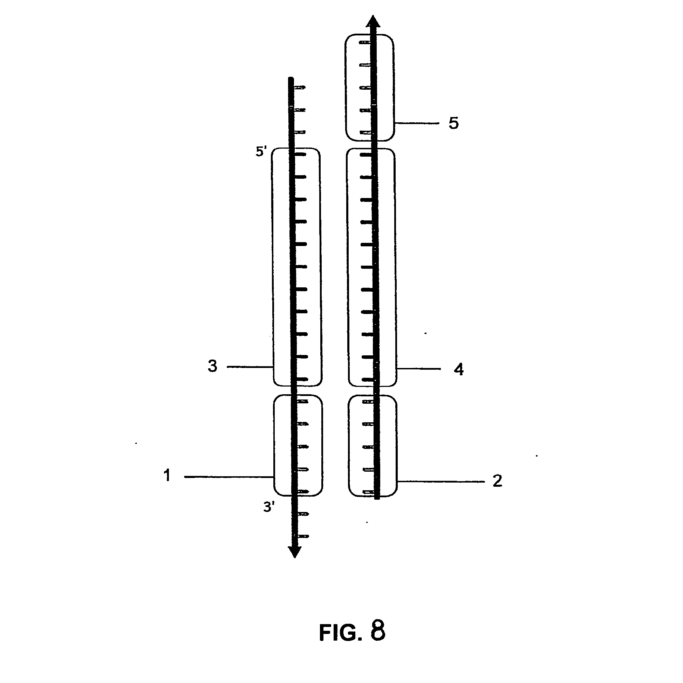

**FIG. 8** is an enlarged schematic illustration of the final, ligated DNA fragment depicted in FIG. 7 showing the various distinct sequence blocks. 1 = complement of the first universal sequence; 2 = first universal sequence; 3,4 = specific sequence block; 5 = complement of the second universal sequence.

### DETAILED DESCRIPTION OF THE INVENTION

The present invention is based on the discovery that the use of two different DNA microarrays for primer synthesis and hybridization, respectively, provides a highly cost-effective way to identify single nucleotides, in particular SNPs, at specific positions in target nucleic acids. Conventional techniques for determining SNPs are still quite expensive and generally do not cover the costs unless a large number of nucleic acid samples are screened for SNPs. For example, the determination of SNPs using a commercially available, tailored DNA microarray, where customized oligonucleotides are directly used as SBE primers, typically requires the analysis of at least about 1000+ nucleic acid samples in order to be cost-efficient. In contrast, the methods of the present invention require the analysis of just about 20+ nucleic acid samples to cover the costs.

The cost benefits of the methods of the present invention are mainly based on the unique way of primer synthesis using a first DNA microarray (synthesis array). The synthesis array includes a plurality of immobilized oligonucleotides, each designed to detect a specific nucleotide (e.g., a SNP). These oligonucleotides are used in the manufacture of primers (SBE primers). The prepared SBE primers can then be extended by a single detectable labeled nucleotide in a template-dependent, polymerase-catalyzed primer extension reaction. The single nucleotide of interest (e.g., a SNP) is identifiable by hybridization of the extended primer on a second DNA array (hybridization array), followed by the detection of the detectable label of the incorporated nucleotide. The unique primer synthesis approach of the present invention using a synthesis array allows one to produce up to 1 x 10⁶ different SBE primers at much lower costs than has been possible until now.

It is believed that the high number or high amount of SBE primers generated and used in the methods of the present invention statistically compensate for nucleotide errors in the oligonucleotides. This provides a cost benefit compared to the primers employed in known commercial techniques for the identification of SNPs that typically have very few synthesis errors but are also relatively costly. Thus, the method of the present invention is economically highly favourable while it at the same time allows for the identification of single point mutations with high specificity and sensitivity.

In a first aspect, the present invention relates to a method for producing single base extension (SBE) primers, the method comprising steps (a) to (e). In step (a), there is provided a first DNA microarray. The first DNA microarray has a plurality of different immobilized DNA oligonucleotides. The oligonucleotides have a particular structure in that each oligonucleotide comprises a specific DNA sequence present in the target nucleic acid. This specific DNA sequence is adjacent to, and immediately upstream of, the nucleotide to be identified.

Preferably, two different oligonucleotides are used for each single nucleotide (e.g., SNP) to be analyzed. In other words, the first DNA microarray preferably has two different oligonucleotides immobilized on its surface for each single nucleotide to be identified. The first oligonucleotide comprises a specific DNA sequence of one of the strands of the target nucleic acid immediately upstream of the single nucleotide to be identified, and the second oligonucleotide comprises a specific DNA sequence of the other strand of the target nucleic acid immediately upstream of the nucleotide complementary to the single nucleotide to be identified. It is noted that it is well possible to carry out the method of the present invention using only one oligonucleotide probe for each single nucleotide to be identified. However, the use of two different oligonucleotide probes increases reliability and provides a higher level of accuracy.

Suitable oligonucleotides for use in the present invention may have a length of up to 90 nucleotides but are usually 30 to 85 nucleotides in length. More typically is a length of between 50 and 85 nucleotides. Preferred oligonucleotides are 50-mers to 80-mers, more preferably 54-mers to 80-mers and especially preferred 54-mers to 60-mers. Typically, the target nucleic acid specific DNA sequence amounts to roughly about half of the entire oligonucleotide sequence. For example, the especially preferred 54-mer to 60-mer oligonucleotides include usually a 26-mer to 32-mer specific sequence.

The indicated length of the oligonucleotides, in particular the length of the target nucleic acid specific sequence (e.g. the mentioned 26-mer to 32-mer sequence), represents an excellent compromise between higher specificity of shorter oligonucleotides (e.g., 25-mer oligonucleotides) and higher sensitivity of longer oligonucleotides (>40-mer oligonucleotides). Furthermore, the oligonucleotides used in the present invention preferably have a so-called "isothermal design", where the oligonucleotide length is varied in an attempt to obtain an approximately constant melting temperature.

The first DNA microarray generally carries between 2,000 and 1,000,000, and typically between 60,000 or 100,000 and 500,000 different oligonucleotides located on the microarray in the form of, for example, DNA spots, areas or zones. The number of identical oligonucleotides on the first DNA microarray depends on the number of single nucleotides (e.g., SNPs) to be analyzed (e.g., 30,000, 90,000, 200,000 or 500,000) and the microarray size. If, for example, 30,000, 90,000 or 200,000 single nucleotides are to be analyzed using a microarray carrying about 1,000,000 oligonucleotide probes, a particular oligonucleotide sequence occurs more than once. However, if 500,000 single nucleotides (e.g., SNPs) are to be analyzed using a microarray of 1,000,000 oligonucleotides and, as described above, two different oligonucleotide sequences are used for each single nucleotide to be analyzed, then each sequence occurs only once.

The support on which the oligonucleotides are immobilized is not particularly limited and may include glass slides or silicon chips. Within the present invention, the oligonucleotides on the DNA microarray may by attached by their 3' ends or by their 5' ends, wherein the oligonucleotides are preferably attached by their 3' ends. Generally, the oligonucleotides are *in situ* synthesized in the 3' to 5' direction or, less common, in the 5' to 3' direction. The type of bonding between the oligonucleotides and the surface of the support is not limited in any way. For example, epoxysilane-coated glass slides may be used for immobilizing the first nucleotide thereon, as known in the art. Specific DNA microarrays designed to suit the needs of the present invention can be obtained from various companies which offer customized DNA microarrays (e.g., Agilent, Roche NimbleGen and Mycroarray).

In step (b), DNA strands that are complementary to the immobilized DNA oligonucle-otides are synthesized using a polymerase enzyme. This step is carried out directly on the first DNA microarray by contacting the array with a reaction mixture, comprising a primer, dNTPs (deoxyribonucleoside triphosphates), a polymerase and a reaction buffer, and incubating the array at a given temperature for a certain period of time. The primer is typically a short, chemically synthesized DNA oligonucleotide. Preferably, the primer has a length of about 8 to 30 nucleotides, more preferably of about 10 to 20 nucleotides, and most preferably of about 12 to 18 nucleotides. Suitable is an incubation for 10 min to 60 min at a temperature between 18°C and 37°C, more typically is a two-step protocol with an incubation for 2 min to 20 min, preferably 5 min to 15 min, at a temperature between 15°C and 30 °C, preferably between 18°C and 25 °C, followed by an incubation for 10 min to 50 min at a temperature between 20 °C and 40°C, preferably between 25 °C and 37°C. Most preferably, the incubation is for 5 min to 15 min at a temperature between 22°C and 25 °C, followed by an incubation for 15 min to 40 min at 35°C to 37 °C.

The dNTPs include all four dNTPs (dATP, dTTP, dGTP, dCTP) in sufficient amounts to allow the polymerase to synthesize the complementary strands. Suitable polymerases include, but are not limited to, T4 polymerase, Klenow fragment and Taq polymerase. The reaction buffer is not particularly limited and may be appropriately selected depending on the specific polymerase and reaction conditions used. Usually, a Tris-HCl buffer, in particular a Tris-HCl buffer including potassium and magnesium acetate, is suitable. Appropriate buffers are known or can be readily determined by a person skilled in the art.

In step (c), the synthesized complementary DNA strands are separated from the first DNA microarray. The separation may be effected by adding a suitable solvent, such as water, to the microarray. The solvent containing the synthesized oligonucleotides may be collected in any suitable vessel, such as a tube. Typically, an amount of about 700 µl water is added to the microarray and collected. The concentration of eluted oligonucleotides in water is usually about 10 to 20 nM. For example, if the used DNA microarray carries about 1 x 10⁶ oligonucleotides, approximately 10 pmol complementary DNA strands are synthesized. The concentration of eluted oligonucleotides in 700 µl water is thus 14 nM.

Although the separated, e.g. eluted, oligonucleotides can be directly used in the next step of the method according to the present invention, the oligonucleotides are preferably purified before further use. The reason is that the solution of the separated oligonucleotides generally has to be concentrated to a smaller volume for use in the PCR of step (d), thereby also concentrating undesirable by-products or contaminants that may interfere with the PCR reaction. Typically, the solution has to be concentrated to a smaller volume of typically about 50 µl for application in the PCR reaction. This means that, after the concentration step, the oligonucleotide concentration is usually between 0.14 µM and 0.28 µM, preferably about 0.20 µM.

The purification of the separated synthesized oligonucleotides can be effected as known in the art, for example by precipitation using isopropanol (1/10 vol 5 M NaCl, 1 vol isopropanol, 1 µl glycogen; 15 min to 2 h at room temperature), followed by centrifugation (10,000 rpm) and washing with ethanol. Alternatively, the precipitation may be carried out using ethanol (2 vol 96% ethanol; 15 min to 2 h at -20°C), followed by centrifugation (14,000 rpm) and washing with 70% ethanol. Another possibility is the purification using nucleic acid purification columns.

In step (d), the separated complementary DNA strands are amplified by PCR (polymerase chain reaction) to produce a PCR product. The amplification of DNA strands by PCR is well known in the art and optimized reaction conditions may be readily determined by a person skilled in the art. The forward and reverse PCR primers are each complementary to and anneal with the 5' terminal parts of the sense and antisense strands. Preferably, the length of the forward and reverse PCR primers is from 8 to 30 nucleotides, more preferably from 10 to 20 nucleotides, and particularly preferred from 12 to 18 nucleotides.

The polymerase used in the PCR reaction may be the same or different to the polymerase used in step (b), provided the polymerase is thermostable. Suitable polymerases include, but are not limited to, DyNAzyme^{™} Hot Start polymerase (Finnzyme), Pushion^{®} (Finnzyme), Taq polymerase (New England Biolabs), and KAPA HiFi™ (Kappa Biosystems). A preferred polymerase is KAPA HiFi™ (Kappa Biosystems).

The PCR product is typically a double-stranded product. However, the present invention is not limited to double-stranded PCR products. Also contemplated herein is a PCR carried out under conditions that result in the preferential amplification of one of the two strands so that the PCR product is single-stranded, predominantly single-stranded or at least partially single-stranded. The generation of a predominantly single-stranded PCR product may be advantageous in that it facilitates the optional isolation/purification of the cleaved DNA single-strands prior to further use as SBE primers.

A suitable PCR reaction mixture may, for example, comprise 20 to 100 µl of the complementary strands obtained in step (c), 0.5 to 3 mM MgCl₂, 1 to 4 mM of each dNTP (e.g., dATP, dTTP, dGTP and dCTP), 150 to 250 µM forward primer and 150 to 250 µM reverse primer, and 1 to 5 Units polymerase, based on a total reaction volume of about 100 µl. The PCR conditions are not particularly limited and the following PCR protocol may be used: incubation at 94°C for 10 min, followed by 10 cycles of 94 °C for 20 sec, 41°C for 10 sec, 42 °C for 10 sec, 43 °C for 10 sec, 44°C for 10 sec, 45 °C for 10 sec, and 72 °C for 20 sec.

Optionally, the obtained PCR product may be purified prior to further use. For example, the PCR product may be run on an agarose gel, cut-out and isolated as known in the art. However, this step can also be omitted, provided the PCR buffer does not exert an inhibiting effect on the restriction enzyme used in the following cleavage step.

In step (e) the PCR product is cleaved immediately adjacent to, and downstream of, the (target nucleic acid) specific DNA sequence discussed above in connection with step (a) to obtain cleaved DNA strands having a 3' end terminating (exactly) one nucleotide before the nucleotide to be identified. The term "cleavage", as used herein, is not intended to be restricted to a particular type of cleavage and includes cleavage of DNA by chemical and enzymatic means.

In a second aspect, the present invention relates to a method for identifying single nucleotides at specific positions in at least one target nucleic acid, the method comprising steps (a) to (e) as described above, followed by steps (f) to (i). Within the present invention, the "single nucleotide to be identified" is preferably a SNP.

In step (f), the primers of step (e) are used as SBE primers and are extended by a single nucleotide by means of hybridizing the SBE primers to at least one target nucleic acid and incubating the formed hybridization complexes of target nucleic acid and SBE primer in the presence of at least one detectably labeled chain-terminating nucleotide and a polymerase to cause incorporation of the detectably labeled chain-terminating nucleotide at the 3' terminus of the SBE primers.

Preferably, the cleaved DNA strands of step (e) are isolated, and thereby purified, prior to their further use as, for example, single base extension (SBE) primers. Methods to isolate DNA are known to a person skilled in the art. Although not required, this isolation step improves binding of the SBE primers to the target nucleic acid and to the antisense oligonucleotides on the second DNA array since it leads to the removal of substances that potentially interfere with hybridization.

The at least one target nucleic acid may be present in any suitable form. For example, the DNA that comprises or consists of the target nucleic acid(s) may be amplified DNA (whole-genome amplified DNA), degraded DNA or any DNA sample containing genomic DNA in non-isolated form, such as body fluids like blood and saliva, histological tissue sections, biopsies, cell sap, and the like.

The SBE reaction can be carried out under reactions known to a person skilled in the art. In brief, a mixture, comprising the SBE primers of step (e), the target nucleic acid(s), a polymerase and at least one ddNTP as detectably labeled chain-terminating nucleotide, may be subjected to multiple SBE thermocycles to result in the incorporation of the at least one ddNTP at the 3' terminus of the SBE primers.

Preferably, the extended SBE primers obtained in step (f) are isolated because unincorporated detectably labeled chain-terminating nucleotides may adsorb on the support of the second DNA microarray, thereby potentially leading to undesirable background signals. In addition, if there is no isolation step, the remaining target DNA may compete with the detectably labeled, extended SBE primers for binding sites of the antisense oligonucleotides on the second DNA microarray.

In step (g), there is provided a second DNA microarray, the second DNA microarray having a plurality of different immobilized DNA antisense oligonucleotides assigned to different respective microarray locations, each of the assigned DNA antisense oligonucleotides being complementary to at least a portion of the different immobilized DNA oligonucleotides on the first DNA microarray. It should be understood that the second DNA microarray can be provided at any time prior to or after any one of steps (a) to (f) and does not have to be carried out after step (f).

Each of the different antisense oligonucleotides on the second DNA microarray is assigned to a different microarray location to allow allocation of the detectable signals originating from the detectable labels of the hybridized oligonucleotides (i.e. the extended SBE primers). The pre-determined location of each antisense oligonucleotide allows the signals originating from the detectable labels to be assigned to a specific antisense oligonucleotide sequence and, thus, to a specific nucleotide at a specific position in the target nucleic acid(s). In general, the assignment of the xylocation on the second DNA microarray to the respective antisense oligonucleotide is saved in a file to thereby conveniently allow the pattern of individual signals to be evaluated and assigned to specific DNA sequences using a computer.

A multiplex microarray format may also be used in accordance with the present invention. Such a format allows the convenient and simultaneous analysis of multiple independent DNA samples. For example, a 8 x 60K microarray enables the simultaneous hybridization of eight different DNA samples. Each "sub"-array includes 60,000 different antisense oligonucleotides. Accordingly, if two different oligonucleotides on the first DNA microarray and thus two different antisense oligonucleotides on the second DNA microarray are used for each single nucleotide (e.g., SNP) to be analyzed, the use of such a multiplex array allows one to analyze 30,000 different single nucleotides.

As noted for the first DNA microarray, the second DNA microarray can be obtained from companies that are specialized in the fabrication of custom-made microarrays. For further details about suitable microarrays, it is referred to the explanations given above in relation to the first DNA microarray which apply *mutatis mutandis* to the second DNA microarray.

In step (h), the extended SBE primers are hybridized to the plurality of different immobilized DNA antisense oligonucleotides assigned to different respective microarray locations. Suitable hybridization conditions are known to a person skilled in the art. Typically, the hybridization is performed in a buffer containing 5 x SSC and 0.1 % SDS and incubated overnight (e.g., 8 to 20 hours) at a temperature between 60 to 65 °C.

Finally, in step (i), the incorporated chain-terminating nucleotides at the 3' terminus of the extended SBE primers are identified by detecting the detectable label of said chain-terminating nucleotides for each of the extended SBE primers hybridized to the different immobilized and assigned DNA antisense oligonucleotides on the second microarray, thereby determining the nucleotide to be identified. In other words, a detectable signal at a particular location on the second DNA microarray allows a specific antisense oligonucleotide sequence (and thus also the respective complementary oligonucleotide sequence) to be assigned to the signal. In addition, the type of signal, for example fluorescence color in case of more than one detectably labeled chain-terminating nucleotide, allows one to determine the type of incorporated, detectably labeled, chain-terminating nucleotide.

In a third aspect, the present invention relates to an alternative method for producing single base extension (SBE) primers comprising steps (a) and (e) as described above and steps (b') to (d'). According to step (b') of the alternative method, the immobilized assigned DNA oligonucleotides are separated from the first DNA microarray prior to synthesis of the complementary strands. This separation step allows, in the following step (c'), the enzymatic synthesis of the complementary strand to be carried out in a suitable reaction vessel, which may lead to additional cost saving potentials and improve ease of use.

Prior to step (d'), the complementary strands obtained in step (c') may optionally be isolated. In step (d'), the oligonucleotide strands are amplified by polymerase chain reaction (PCR) to produce a PCR product. With regard to the PCR, it is referred to the explanations set out above in relation to step (d) of the first alternative method. Furthermore, the obtained PCR product may be purified as described above before further use.

In a fourth aspect, the present invention relates to an alternative method for identifying single nucleotides at specific positions in at least one target nucleic acid. The method corresponds to the method according to the second aspect, except that steps (b) to (d) are replaced by steps (b') to (d'). In other words, steps (a) and (e) to (i) described above are identical in the methods according to the second and fourth aspect of the present invention.

In accordance with the present invention, the methods according to the first to fourth aspects utilize a first DNA microarray where the immobilized DNA oligonucleotides comprise a specific DNA sequence present in the target nucleic acid immediately upstream of the nucleotide to be identified, the complement of a first universal sequence attached to the 3' end of said specific DNA sequence, and a second universal sequence different from the first universal sequence attached to the 5' end of said specific DNA sequence. Hence, each of the enzymatically synthesized complementary DNA strands comprises the complement of said specific DNA sequence, the first universal sequence attached to the 5' end of said complement of the specific DNA sequence, and the complement of the second universal sequence attached to the 3' end of said complement of the specific DNA sequence.

In a fifth aspect, the present invention relates to another alternative method for producing single base extension (SBE) primers comprising steps (a) and (e) as described above and steps (b"), (c1"), (c2") and (d"). According to step (b") of this alternative method, DNA strands complementary to the immobilized DNA oliaonucleotides are enzymatically synthesized, on the first DNA microarray, to form an immobilized, double-stranded DNA fragment. The first DNA microarray is then usually rinsed with a suitable solvent, for example 10 mM Tris-HCl, pH 7.5, to remove dNTPs, polymerase, buffer and primers.

Next, in step (c1") the double-stranded DNA fragment is cleaved off from the first DNA microarray. Generally, a restriction enzyme is used for cleavage that generates cohesive ("sticky") ends with either 5' overhangs or 3' overhangs and/or blunt ends. Preferred are cohesive ends, in particular cohesive ends with 3' overhangs. Suitable reaction conditions for restriction digestion are known to a person skilled in the art.

Optionally, the cleaved off double-stranded DNA fragments may be purified in accordance with conventional procedures to remove any compounds that potentially interfere with the subsequent ligation reaction. For example, the double-stranded DNA fragments may be run on an agarose gel, cut out and isolated via the NucleoSpin Gel and PCR Clean-up kit (Machery & Nagel).

After that, in step (c2"), the cleaved off double-stranded DNA fragment is ligated to one or two linkers (one at each end) to obtain a ligated double-stranded DNA fragment. The double-stranded linkers used in the ligation reaction are typically formed by hybridization of two primers as known in the art. Usually, one of the double-stranded linkers contains a strand consisting of the first or second universal sequence. The 5' end of one primer of each linker which is to be ligated to the double-stranded DNA fragments is preferably phosphorylated at the 5' end to facilitate ligation. After ligation, the ligation product is preferably purified as known in the art, for example as described above in connection with step (c1").

Then, in step (d"), the ligated double-stranded DNA fragment is amplified by polymerase chain reaction (PCR) to produce a PCR product including a strand having the complement of a first universal sequence at one end and a second universal sequence at the other end. For the PCR, the same reaction conditions as described above with respect to step (d) can be used.

According to a preferred embodiment of the methods according to the fifth aspect of the present invention, the DNA oligonucleotides are immobilized through their 3' ends on the first DNA microarray. Further, the DNA oligonucleotides immobilized on the first DNA microarray preferably comprise a specific DNA sequence present in the target nucleic acid immediately upstream of the nucleotide to be identified and the complement of a universal sequence attached to the specific DNA sequence, preferably to the 3' end of the specific DNA sequence. Hence, each of the enzymatically synthesized complementary DNA strands comprises the complement of said specific DNA sequence and the first universal sequence attached thereto, preferably attached to its 5' end.

The ligation of linkers to double-stranded DNA fragments having only one distinct universal sequence may be favorable compared to the additional inclusion of another universal sequence in oligonucleotides on the microarray as in the case of the method according to the first and third aspects of the present invention. Error rates of oligonucleotides on the DNA microarray increase progressively with synthesis cycles. For example, 3' regions of oligonucleotides that are synthesized in 3' to 5' orientation on the DNA microarray are of higher quality than 5' regions. A high rate of synthesis errors in the 5' universal sequences could reduce the efficiency of the PCR amplification. The ligation of linkers would avoid this and increase the PCR yields. In addition, the alternative method of the fifth aspect of the present invention obviates the step of separating the synthesized, complementary strand oligonucleotides from the first DNA microarray, and the subsequent (preferred) clean-up step.

In a sixth aspect, the present invention relates to another alternative method for identifying single nucleotides at specific positions in at least one target nucleic acid. The method corresponds to the method according to the second aspect, except that steps (b), (c), and (d) are replaced by steps (b"), (c1") and (c2"), and (d"). In other words, steps (a) and (e) to (i) described above are the same in the methods according to the second, fourth and sixth aspect of the present invention.

In accordance with a preferred embodiment of the methods according to the present invention, the PCR in step (d) is preferably carried out using the first and second universal sequences as reverse and forward PCR primers. The second universal sequence preferably contains a biotin group or derivative thereof at its 3' end, which facilitates isolation by streptavidin-binding, and the first universal sequence preferably does not include a biotin group or derivative thereof. A particularly preferred biotin derivative is desthiobiotin.

According to another preferred embodiment of the methods according to the present invention, the cleavage of step (e) is effected using a restriction endonuclease. The cleavage site is selected so that the complement of the first universal sequence is removed from the single strands so as to generate SBE primers, the 3' end of the SBE primers terminating one nucleotide before the nucleotide to be identified.

As mentioned above, the SBE primers obtained in step (e) preferably contain a biotin group or derivative thereof at their 5' ends, which has been incorporated by using the second universal sequence carrying a biotin group or derivative thereof at the 5' end as PCR primer in step (d) and (d'), respectively. Thus, in a preferred embodiment of the present invention, the isolation of the SBE primers prior to the SBE extension reaction of step (f) is carried out by non-covalently binding the biotin group or derivative thereof to a streptavidin-coated support, e.g. streptavidin-coated magnetic beads, and separating the resulting streptavidin-biotin complex.

According to a further preferred embodiment of the methods according to the present invention, the single nucleotide primer extension of step (f) is carried out in the presence of at least one, e.g. 1, 2, 3 or 4, detectably labeled chain-terminating nucleotide selected from the group consisting of ddATP, ddTTP, ddUTP, ddCTP and ddGTP, but in the absence of dATP, dTTP, dUTP, dCTP and dGTP. Preferably, the detectable label of the at least one chain-terminating nucleotide is a fluorophore. Preferably, two, three or four of the mentioned ddNTPs are labeled with fluorophores of different colors. This makes it possible to reliably analyze not only biallelic SNPs but also tri-and tetraallelic SNPs. Within the present invention, it is particularly preferred to use four different ddNTPs (i.e., ddATP, ddTTP, ddCTP, ddGTP and/or ddATP, ddUTP, ddCTP, ddGTP), each labeled with a different label, for example different fluorophores. Examples of such fluorescence-labeled ddNTPs include, but are not limited to, 7-Propargylamino-7-deaza-ddGTP-Cy5, 7-Propargylamino-7-deaza-ddATP-Cy3-,5-Propargylamino-ddCTP-ATTO-488,5-Pro-pargylamino-ddUTP-Texas Red.

In a seventh aspect, the present invention relates to a method for determining the genotype of an organism at one or more single nucleotide polymorphic sites, which comprises identifying the nucleotide present at the single nucleotide polymorphic site, using the methods of identifying single nucleotides described herein, thereby determining the genotype of the organism at the one or more single nucleotide polymorphic sites.

The organism may include any unicellular or multicellular organism and is preferably a human, an animal, a plant or a microorganism. As used herein, the term "animal" refers to any animal excluding human beings. The term "microorganism" includes bacteria, fungi, algae and protozoons.

In an eighth aspect, the present invention relates to a method for predicting a monogenic or complex trait of a human, animal or plant by determining the genotype of an organism at one or more single nucleotide polymorphic sites, using the methods of identifying single nucleotides described herein, the one or more single nucleotide polymorphic sites being associated with or indicative of a specific monogenic or complex trait of the human, animal or plant.

As used herein, a "monogenic trait" is intended to mean a trait that is strongly influenced by variation within a single gene. The term "complex trait", as used herein, refers to any phenotype that does not exhibit classic Mendelian recessive or dominant inheritance attributable to a single gene locus. A "complex trait" is usually the result from variation within multiple genes. It may also be influenced by environmental factors. Examples of complex traits that are of commercial interest include taste, growth, yield, appearance, tolerance to environmental stresses and the like.

The method for predicting a monogenic or complex trait is anticipated to significantly shorten the development time for obtaining improved plants and animals. Thus, the present method allows breeders to specifically select plants and animals with respect to the desired complex trait. As a result, time and costs required for the desired breeding success can be significantly decreased.

In a ninth aspect, the present invention pertains to a method for assessing the genetic similarity between a target organism or a target population of organisms and a reference organism and/or a reference population of organisms, which comprises identifying the nucleotide at a single nucleotide polymorphic site, using the methods for identifying single nucleotides described herein, of the target organism or target population of organisms and a reference organism and/or a reference population of organisms, and determining the genetic similarity based on the single nucleotide at the polymorphic site.

As used herein, the term "target population" refers to a group of individual organisms. The organisms are typically more or less related individual organisms and can, for example, be identified by genetic traits, phylogenetic relationship, geographic location, and the like. The size of the target population can vary from ten or hundred to millions of organism. In addition, the term "reference population", as used herein, refers to a group of individual control organisms which are usually selected to approximate as far as possible to the target population or target organism. Further, the organism may include any unicellular or multicellular organisms as defined above.

In a tenth aspect, the present invention concerns a method for identifying at least one single nucleotide polymorphic site as a diagnostic disease marker, which comprises identifying a nucleotide at the at least one single nucleotide polymorphic site, using the methods for identifying single nucleotides described herein, of one or more healthy organisms and one or more diseased organisms, and determining whether there is a difference at the at least one single nucleotide polymorphic site of the one or more healthy organisms and the one or more diseased organisms, a difference being indicative of the suitability of the at least one single nucleotide polymorphic site as a diagnostic marker.

In an eleventh aspect, the present invention relates to a method for diagnosing a disease in a subject, comprising identifying a single nucleotide at one or more single nucleotide polymorphic sites, using the methods for identifying single nucleotides described herein, in a nucleic acid sample of the subject, and determining whether the identified one or more nucleotides correspond to a single base polymorphism associated with said disease.

In a twelfth and last aspect, the present invention provides a kit for identifying single nucleotides at specific positions in at least one target nucleic acid, comprising a first DNA microarray and a second DNA microarray as described herein. The kit may further comprise at least one, preferably two, more preferably three, and most preferably four detectably labeled chain-terminating nucleotide(s) selected from the group consisting of ddATP, ddTTP, ddUTP, ddCTP and ddGTP. Preferably, the detectable label is a fluorophor.

The present invention will now be further illustrated by the following examples.

### EXAMPLES

### Example 1

The following example shows that the use of a first synthesis array and a second hybridization array in accordance with the present invention is a specific and sensitive method for simultaneously identifying thousands of single nucleotides (e.g, SNPs) at specific positions in target nucleic acids in a cost-efficient manner.

### 1. Desgin of the first microarray

With reference to **FIG. 1**, the double-strand of a target nucleic acid may contain a nucleotide (base), e.g. a SNP, to be interrogated in one of the two strands (see, e.g., the nucleotide highlighted by the lower arrow). The other strand includes the complementary nucleotide (base) (see, e.g., the nucleotide highlighted by the upper arrow). The specific DNA sequence immediately before the nucleotide to be interrogated in the 5' direction of the lower strand, and the specific DNA sequence immediately after the complementary nucleotide in the 5' direction of the upper DNA strand is incorporated in two different oligonucleotides of the first microarray (see **FIG. 1**; the specific nucleotide sequences are denoted by solid black vertical lines).

Hence, the specific DNA sequences of the exemplary target nucleic acid are each adjacent to, and immediately upstream of (i.e. on the 5' side), the nucleotide to be identified and the respective complementary nucleotide. This is, in this exemplary embodiment, each nucleotide to be interrogated (e.g., SNP) is represented by two oligonucleotides.

As shown in **FIG. 2**, each of the two ends of the aforementioned sequence blocks are appended with two distinct sequences. The 3' end of the specific DNA sequence is attached to the complement of a first universal sequence, and the 5' end is attached to a second universal sequence. The first and the second universal sequences are not identical.

### 2. Design of the second microarrray

For each oligonucleotide on the first microarray there is a corresponding antisense oligonucleotide on the second microarray. Considering the oligonucleotide depicted in FIG. 2 consisting of, from the 5' to the 3' end, the second universal sequence, a specific sequence block present in the target nucleic acid, and the complement of the first universal sequence, the corresponding antisense oligonucleotide of the second microarray is the complement thereof but lacks the first universal sequence. It should be understood that both the oligonucleotide of the first array and the antisense oligonucleotide of the second array are only exemplary sequences which are not intended to limit the invention in any way.

### 3. Microarray-based synthesis of oligonucleotides

The microarray-based synthesis of oligonucleotides is the first step in the SBE primer synthesis (see sections 3 to 5 of this example). An overview of the synthesis of the SBE primers is given in **FIG. 3** in order to ease understanding of the process.

The first microarray as described above was used for the synthesis of strands complementary to the immobilized oligonucleotides (see **FIG. 3A**) by adding 700 µl reaction mixture, containing 500 nM of a 14-mer primer (first universal sequence), 10 µM dNTPs, 10 Units T4 polymerase (Epicentre) and 1x reaction buffer (33 mM Tris-acetate (pH 7.5), 66 mM potassium acetate, 10 mM magnesium acetate, and 0.5 mM DTT), to the first microarray and incubating the array for 5 min at 22°C, followed by incubation for 15 min at 37°C (see **FIG. 3B**).

For isolation of the synthesized oligonucleotides, 700 µl water was added to the microarray, incubated for 5 min at 95°C and collected in a tube (see **FIG. 3C**). This step is repeated twice. For clean-up of the 60-mer oligonucleotides, the NucleoSpin Gel and PCR Clean-up kit (Machery & Nagel) were used with the binding buffer NTC (Machery & Nagel) instead of the binding buffer provided with the kit.

### 4. Amplification

Synthesis of the complementary strands to the strands obtained (see section 3) and amplification of the double-stranded DNA fragments were carried out by PCR in 100 µl reaction volume with the following specifications: 15 µl clean-up product from the previous step 3, 1.5 mM MgCl₂, 2.5 mM dNTPs, 200 µM first universal sequence as a primer, 200 µM second universal sequence modified with desthiobiotin at the 5' terminus as a second primer, and 2.5 Units DyNAzyme Hot Start polymerase (Finnzyme) (see **FIG. 3D**).

After incubation at 94°C for 10 min, there were carried out 10 cycles of 94°C for 20 sec, 41°C for 10 sec, 42 °C for 10 sec, 43°C for 10 sec, 44 °C for 10 sec, 45°C for 10 sec, and 72°C for 20 sec. For clean-up, the resulting PCR product is run on an agarose gel, cut out and isolated via the NucleoSpin Gel and PCR Clean-up kit (Machery & Nagel).

### 5. Cleavage and recovery of primers

In order to remove the first universal sequence, the double-stranded product was subjected to restriction digestion (see **FIG. 3E**). The reaction was carried out using 100 Units Bts I in 20 mM Tris-acetate, 50 mM potassium acetate, 10 mM magnesium acetate, 1 mM DTT, and incubating for 16 h at 55°C.

The isolation of the desired desthiobiotinylated strands was carried out by streptavidin coupled to magnetic beads (Dynabeads M-270 Streptavidin by Life Technologies) (see **FIG. 3F-H**). Binding of the restriction digested products to 10 mg of Dynabeads and washing was carried out according to the manufacture's protocol using 2xB&W buffer (10 mM Tris-HCl (pH 7.5), 1 mM EDTA, 2 M NaCl) (see **FIG. 3F**).

Denaturation of the double-strand was performed by adding formamide to obtain a 75% formamide solution. Separation of the desthiobiotinylated strands coated beads was achieved by placing the tube into a magnetic stand for 3 min (see **FIG. 3G**). After three washing steps; the desthiobiotinylated strands were recovered in 100 µl water by incubation for 10 min at 70°C (see **FIG. 3H**).

### 6. Single base extension (SBE) of primers

SBE reactions were carried out in 50 µl reactions using 1 µl of the product from the previous step (see section 5), 20 µg of whole-genome amplified genomic DNA, 2 Units of Thermo Sequenase (GE Healthcare), 52 mM Tris-HCl (pH 9.5), 6.5 mM MgCl₂, and 7,5 µM of each of the following dye-modified ddNTPs: 7-propargylamino-7-deaza-ddGTP-Cy5, 7-propargylamino-7-deaza-ddATP-Cy3, 5-propargylamino-ddCTP-ATTO-488, 5-propargylamino-ddUTP-Texas Red. Extension reactions were carried out on a peqS-TAR 2X Gradient Thermocycler (PeqLab) with 1 cycle at 96°C for 3 min and 40 cycles (96 °C for 20 sec, ramp from 75 °C to 60 °C with 0.25 °C per sec, 60 °C for 60 sec).

### 7. Clean-up of primers

The clean-up of the extension-labeled desthiobiotinylated primers was carried out by streptavidin coupled to magnetic beads as described above (see section 5), with the following differences: 0.1 mg of Dynabeads, no formamide addition, recovery in 10 µl water.

### 8. Hybridization, wash and scan

The purified labeled primers were then hybridized to the second microarray (see section 2). For hybridization and washing, Agilent's manual for miRNA analysis was used including all required reagents. Briefly, for an 8-plex hybridization, 10x GE Blocking Agent, 2x Hi-RPM Hybridization Buffer and labeled primer in 45 µl were incubated at 100 °C for 5 min, added to the second microarray and transferred to the hybridization chamber (Agilent). The hybridization was then carried out in an hybridization oven (Agilent) at 60 °C under rotation at 20 rpm for 20 hours.

The disassembly of the hybridization chamber is carried out in wash buffer 1 (1X SSC buffer (1X = 0.150 M NaCl, 0.015 M sodium citrate), 0.2% SDS, 1 mM DTT). Afterwards, the microarray was placed in wash buffer 1 for 5 min at room temperature, followed by a wash in wash buffer 2 (0.1X SSC buffer, 0.2% SDS, 1 mM DTT) at 37 °C for 5 min. After air-drying, the microarray is placed in a scanner that is sensitive to fluorescent light (GenePix 4400A Scanner by Molecular Devices) and a 4-color 16-bit TIF image was taken.

The picture or pattern of fluorescent signals visible in the TIF image was then subjected to data analysis to determine the single nucleotides (SNPs) at the specific position in the target nucleic acid. In short, the signal intensities were extracted from the recorded TIF image using the software supplied with the scanner (Genepix). Then, the signal intensities were processed and assigned to the incorporated nucleotide and the respective SNP analyzed. The processing can be carried out using commercially available software. In the context of this example, however, the processing was performed using a proprietary software developed by the present inventors that is briefly discussed below.

The proprietary software first determines various key quality indicators from data obtained from artificial oligonucleotides added at different stages of the process (e.g., before the SBE reaction and prior to the hybridization on the second DNA microarray). These key quality indicators are in turn used for calculating treshhold values, which allow the signals to be rated as being noise or a positive signal. Second, the used array design is read in to allow for the assignment of a particular array location to a specific DNA sequence. Third, the two signals for the forward strand and the reverse strand are determined for each color (red, green, blue and yellow). Fourth, it is verified whether the signal combination is plausible, e.g. homozygous A-T and G-C (otherwise mixed colors are detected by means of reference oligonucleotides, half of which are, for example, green labeled with the other half being red labeled). Fifth, provided step four was successful, the respective genotype (A, T, G, C) is assigned.

### Example 2

This example illustrates the ligation-based, alternative method of the present invention for identifying single nucleotides at specific positions in at least one target nucleic acid.

### 1. Design of the first microarray

The design of the first microarray corresponds to that of Example 1, except that the oligonucleotides immobilized on the first microarray contain only one "universal" sequence, i.e. the complement of a universal sequence. The structure of the oligonucleotides is schematically shown in **FIG. 4****.**

As can be seen, the oligonucleotides consist of a specific DNA sequence and the complement of a single universal sequence attached to the 3' end of the specific DNA sequence. The specific DNA sequence is designed as explained above in connection with Example 1 and corresponds to the sequence adjacent to, and immediately upstream of, the nucleotide to be identified (e.g., a SNP). Further, like in Example 1, each nucleotide to be interrogated is represented by two oligonucleotides.

### 2. Design of the second microarray

The design of the second microarray is identical to that described in connection with Example 1.

### 3. Microarray-based synthesis of oligonucleotides

In a first step, complementary strands to the immobilized oligonucleotides were synthesized by using a polymerase and the universal sequence as a primer (see FIG. 5). The reaction conditions were identical to those of Example 1, except that a 14-mer primer having a restriction enzyme cleavage site was used.

After the second (complementary) strand synthesis (see **FIG. 5**, illustration on the right), the microarray was rinsed with 10 mM Tris-HCl, pH 7.5 at room temperature to remove dNTPs, polymerase, buffer and primers.

### 4. Cleavage of double-strands

The double-stranded DNA fragments are then cleaved off from the support (e.g., glass slide) by restricted digestion (see **FIG. 6**). The cleavage reaction was carried out by incubation with 100 Units BbvCl in 20 mM Tris-acetate, 50 mM potassium acetate, 10 mM magnesium acetate, 1 mM DTT, pH 7.9 for 16 h at 37°C. The restriction enzyme is chosen to produce either a 3' overhang or a 5' overhang (see right part of FIG. 6) to facilitate linker ligation. For the purpose of the instant example, the cleaved off DNA fragment is left with a 3' overhang which, however should not be construed to limit the invention in any way.

After cleavage, the DNA fragments were collected and kept in solution. For clean-up, the double-stranded DNA fragments were run on an agarose gel, cut out and isolated via the NucleoSpin Gel and PCR Clean-up kit (Machery & Naqel).

### 5. Linker ligation

Next, the cleaved DNA fragments were ligated with linkers (see **FIG. 7**). Preferably, each end of the DNA fragment is ligated to distinct linkers (see middle drawing of FIG. 7). One linker is ligated to the end of the double-stranded DNA fragment that represents the former 5' end of the oligonucleotide. This linker is ligated to the blunt end of the cleaved DNA fragment and, thus, also has a blunt end for attachment to the DNA fragment. The other linker has two cohesive ("sticky") ends. Alternatively, only one linker (i.e. said linker with a blunt end) may be used for ligation (see right drawing of FIG. 7). The 5' end of one strand of each linker is generally phosphorylated.

For linker ligation, 200 pmol of each linker were mixed with 10 pmol double-stranded DNA fragments. The reaction was carried out using 2,000 Units T4 ligase in 20 mM Tris-acetate, 50 mM potassium acetate, 10 mM magnesium acetate, 1 mM DTT, 1 mM ATP, pH 7.9 for 16 h at 16°C. Upon completion of the reaction, T4 ligase was heat-inactivated by incubating the reaction mixture for 20 min at 65°C.

The final DNA fragment after ligation is shown in the right image of FIG. 7 and consists of a specific sequence block in the middle, flanked by two universal but distinct sequence blocks each having a 3' overhang on both ends. An overview of the various sequence blocks of the final DNA fragment after ligation is shown in **FIG. 8****.**

### 6. Amplification, cleavage and recovery of primers, single base extension (SBE) of primers, clean-up of primers, and hybridization/wash/scan

These steps were carried out in the same manner as described above in relation to Example 1 (see sections 4 to 8). This is, the ligated double-stranded DNA fragment is subjected to PCR amplification using forward and reverse primers corresponding to the first and second universal sequences (see FIG. 8), followed by the steps as outlined above in connection with Example 1.

## Claims

1. A method for producing single base extension (SBE) primers, the method comprising the steps of:
(a) providing a first DNA microarray, the first DNA microarray having a plurality of different immobilized DNA oligonucleotides assigned to different respective microarray locations, each of the immobilized DNA oligonucleotides comprising a specific DNA sequence present in the target nucleic acid immediately upstream of the nucleotide to be identified;
(b) enzymatically synthesizing, on the first DNA microarray, DNA strands complementary to the immobilized DNA oligonucleotides;
(c) separating the complementary DNA strands synthesized in step (b) from the first DNA microarray;
(d) amplifying the separated complementary DNA strands by polymerase chain reaction (PCR) to produce a PCR product;
(e) cleaving the PCR product immediately adjacent to, and downstream of, said specific DNA sequence to obtain cleaved DNA strands having a 3' end terminating one nucleotide before the nucleotide to be identified, the cleaved DNA strands being suited for use as single base extension (SBE) primers.

2. A method for identifying single nucleotides at specific positions in at least one target nucleic acid, the method comprising the steps of:
(a) providing a first DNA microarray, the first DNA microarray having a plurality of different immobilized DNA oligonucleotides assigned to different respective microarray locations, each of the immobilized DNA oligonucleotides comprising a specific DNA sequence present in the target nucleic acid immediately upstream of the nucleotide to be identified;
(b) enzymatically synthesizing, on the first DNA microarray, DNA strands complementary to the immobilized DNA oligonucleotides;
(c) separating the complementary DNA strands synthesized in step (b) from the first DNA microarray;
(d) amplifying the separated complementary DNA strands by polymerase chain reaction (PCR) to produce a PCR product;
(e) cleaving the PCR product immediately adjacent to, and downstream of, said specific DNA sequence to obtain cleaved DNA strands having a 3' end terminating one nucleotide before the nucleotide to be identified, the cleaved DNA strands being suited for use as single base extension (SBE) primers;
(f) extending the SBE primers of step (e) by a single nucleotide by means of hybridizing the SBE primers to said at least one target nucleic acid and incubating the formed hybridization complexes of target nucleic acid and SBE primer in the presence of at least one detectably labeled chain-terminating nucleotide and a polymerase to cause the incorporation of the detectably labeled chain-terminating nucleotide at the 3' terminus of the SBE primers;
(g) providing a second DNA microarray, the second DNA microarray having a plurality of different immobilized DNA antisense oligonucleotides assigned to different respective microarray locations, each of the assigned DNA antisense oligonucleotides being complementary to at least a portion of said different immobilized DNA oligonucleotides of the first DNA microarray;
(h) hybridizing the extended SBE primers to the different immobilized DNA antisense oligonucleotides;
(i) identifying the incorporated chain-terminating nucleotides at the 3' terminus of the extended SBE primers by detecting the detectable label of said chain-terminating nucleotides for each of the extended SBE primers hybridized to the different immobilized and assigned DNA antisense oligonucleotides of the second microarray, thereby determining the nucleotide to be identified.

3. A method for producing single base extension (SBE) primers, the method comprising the steps of:
(a) providing a first DNA microarray, the first DNA microarray having a plurality of different immobilized DNA oligonucleotides assigned to different respective microarray locations, each of the assigned DNA oligonucleotides comprising a specific DNA sequence present in the target nucleic acid immediately upstream of the nucleotide to be identified;
(b') separating the immobilized assigned DNA oligonucleotides from the first DNA microarray;
(c') enzymatically synthesizing, in a reaction vessel, DNA strands complementary to the separated DNA oligonucleotides;
(d') amplifying the synthesized complementary DNA strands by polymerase chain reaction (PCR) to produce a PCR product;
(e) cleaving the PCR product immediately adjacent to, and downstream of, said specific DNA sequence to obtain cleaved DNA strands having a 3' end terminating one nucleotide before the nucleotide to be identified, the cleaved DNA strands being suited for use as single base extension (SBE) primers.

4. A method for identifying single nucleotides at specific positions in at least one target nucleic acid, the method comprising the steps of:
(a) providing a first DNA microarray, the first DNA microarray having a plurality of different immobilized DNA oligonucleotides assigned to different respective microarray locations, each of the assigned DNA oligonucleotides comprising a specific DNA sequence present in the target nucleic acid immediately upstream of the nucleotide to be identified;
(b') separating the immobilized assigned DNA oligonucleotides from the first DNA microarray;
(c') enzymatically synthesizing, in a reaction vessel, DNA strands complementary to the separated DNA oligonucleotides;
(d') amplifying the synthesized complementary DNA strands by polymerase chain reaction (PCR) to produce a PCR product;
(e) cleaving the PCR product immediately adjacent to, and downstream of, said specific DNA sequence to obtain cleaved DNA strands having a 3' end terminating one nucleotide before the nucleotide to be identified, the cleaved DNA strands being suited for use as single base extension (SBE) primers;
(f) extending the SBE primers of step (e) by a single nucleotide by means of hybridizing the SBE primers to said at least one target nucleic acid and incubating the formed hybridization complexes of target nucleic acid and SBE primer in the presence of at least one detectably labeled chain-terminating nucleotide and a polymerase to cause the incorporation of the detectably labeled chain-terminating nucleotide at the 3' terminus of the SBE primers;
(g) providing a second DNA microarray, the second DNA microarray having a plurality of different immobilized DNA antisense oligonucleotides assigned to different respective microarray locations, each of the assigned DNA antisense oligonucleotides being complementary to at least a portion of said different immobilized DNA oligonucleotides of the first DNA microarray;
(h) hybridizing the extended SBE primers to the different immobilized DNA antisense oligonucleotides;
(i) determining the incorporated chain-terminating nucleotides at the 3' terminus of the extended SBE primers by detecting the detectable label of said chain-terminating nucleotides for each of the extended SBE primers hybridized to the different immobilized and assigned DNA antisense oligonucleotides of the second microarray, thereby determining the nucleotide to be identified.

5. A method for producing single base extension (SBE) primers, the method comprising the steps of:
(a) providing a first DNA microarray, the first DNA microarray having a plurality of different immobilized DNA oligonucleotides assigned to different respective microarray locations, each of the assigned DNA oligonucleotides comprising a specific DNA sequence present in the target nucleic acid immediately upstream of the nucleotide to be identified;
(b") enzymatically synthesizing, on the first DNA microarray, DNA strands complementary to the immobilized DNA oligonucleotides to form an immobilized, double-stranded DNA fragment;
(c1") cleaving off the double-stranded DNA fragment from the first DNA microarray;
(c2") ligating one linker or two linkers, one at each end, to the cleaved off double-stranded DNA fragment to obtain a ligated double-stranded DNA fragment;
(d") amplifying the ligated double-stranded DNA fragment by polymerase chain reaction (PCR) to produce a PCR product including a strand having the complement of a first universal sequence at one end and a second universal sequences at the other end;
(e) cleaving the PCR product immediately adjacent to, and downstream of, said specific DNA sequence to obtain cleaved DNA strands having a 3' end terminating one nucleotide before the nucleotide to be identified, the cleaved DNA strands being suited for use as single base extension (SBE) primers;

6. A method for identifying single nucleotides at specific positions in at least one target nucleic acid, the method comprising the steps of:
(a) providing a first DNA microarray, the first DNA microarray having a plurality of different immobilized DNA oligonucleotides assigned to different respective microarray locations, each of the assigned DNA oligonucleotides comprising a specific DNA sequence present in the target nucleic acid immediately upstream of the nucleotide to be identified;
(b") enzymatically synthesizing, on the first DNA microarray, DNA strands complementary to the immobilized DNA oligonucleotides to form an immobilized, double-stranded DNA fragment;
(c1") cleaving off the double-stranded DNA fragment from the first DNA microarray;
(c2") ligating one linker or two linkers, one at each end, to the cleaved off double-stranded DNA fragment to obtain a ligated double-stranded DNA fragment;
(d") amplifying the ligated double-stranded DNA fragment by polymerase chain reaction (PCR) to produce a PCR product including a strand having the complement of a first universal sequence attached to one end and a second universal sequence attached to the other end;
(e) cleaving the PCR product immediately adjacent to, and downstream of, said specific DNA sequence to obtain cleaved DNA strands having a 3' end terminating one nucleotide before the nucleotide to be identified, the cleaved DNA strands being suited for use as single base extension (SBE) primers;
(f) extending the SBE primers of step (e) by a single nucleotide by means of hybridizing the SBE primers to said at least one target nucleic acid and incubating the formed hybridization complexes of target nucleic acid and SBE primer in the presence of at least one detectably labeled chain-terminating nucleotide and a polymerase to cause the incorporation of the detectably labeled chain-terminating nucleotide at the 3' terminus of the SBE primers;
(g) providing a second DNA microarray, the second DNA microarray having a plurality of different immobilized DNA antisense oligonucleotides assigned to different respective microarray locations, each of the assigned DNA antisense oligonucleotides being complementary to at least a portion of said different immobilized DNA oligonucleotides of the first DNA microarray;
(h) hybridizing the extended SBE primers to the different immobilized DNA antisense oligonucleotides;
(i) determining the incorporated chain-terminating nucleotides at the 3' terminus of the extended SBE primers by detecting the detectable label of said chain-terminating nucleotides for each of the extended SBE primers hybridized to the different immobilized and assigned DNA antisense oligonucleotides of the second microarray, thereby determining the nucleotide to be identified.

7. The method of any one of claims 1 to 4, wherein, in step (a), each of the immobilized DNA oligonucleotides comprises a specific DNA sequence present in the target nucleic acid immediately upstream of the nucleotide to be identified, the complement of a first universal sequence attached to the 3' end of said specific DNA sequence, and a second universal sequence different from the first universal sequence attached to the 5' end of said specific DNA sequence,and
wherein each of the enzymatically synthesized complementary DNA strands comprises the complement of said specific DNA sequence, the first universal sequence different from said second universal sequence attached to the 5' end of said complement of the specific DNA sequence, and the complement of the second universal sequence attached to the 3' end of said complement of the specific DNA sequence.

8. The method of any one of claims 1 to 7, wherein the PCR in step (d) is carried out using the first and second universal sequences as reverse and forward PCR primers, the primer of the second universal sequence containing a biotin group or derivative thereof at its 3' end and the primer of the first universal sequence lacking a biotin group or derivative thereof, and/or
wherein, in step (e), the cleavage is effected using a restriction endonuclease, the cleavage resulting in the removal of the complement of the first universal sequence so as to generate SBE primers, the 3' end of the SBE primers terminating one nucleotide before the nucleotide to be identified.

9. The method of any one of claims 1 to 8, wherein, in step (f), the single nucleotide primer extension is carried out in the presence of at least one detectably labeled chain-terminating nucleotide selected from the group consisting of ddATP, ddTTP, ddUTP, ddCTP and ddGTP, but in the absence of dATP, dTTP, dUTP, dCTP and dGTP.

10. A method for determining the genotype of an organism at a single nucleotide polymorphic site, which comprises identifying the nucleotide present at the single nucleotide polymorphic site according to the method of any one of claims 1 to 9, thereby determining the genotype of the organism at the single nucleotide polymorphic site.

11. A method for predicting a monogenic or complex trait of a human, animal or plant by determining the genotype of an organism at one or more single nucleotide polymorphic sites according to the method of any one of claims 1 to 9, the one or more single nucleotide polymorphic sites being associated with or indicative of a specific monogenic or complex trait of the human, animal or plant.

12. A method for assessing the genetic similarity between a target organism or a target population of organisms and a reference organism and/or a reference population of organisms, which comprises identifying the nucleotide at a single nucleotide polymorphic site according to the method of any one of claims 1 to 9 of the target organism or target population of organisms and a reference organism and/or a reference population of organisms, and determining the genetic similarity based on the single nucleotide at the polymorphic site.

13. A method for identifying at least one single nucleotide polymorphic site as a diagnostic disease marker, which comprises identifying a nucleotide at the at least one single nucleotide polymorphic site according to the method of any one of claims 1 to 9 of one or more healthy organisms and one or more diseased organisms, and determining whether there is a difference at the at least one single nucleotide polymorphic site of the one or more healthy organisms and the one or more diseased organisms, a difference being indicative of the suitability of the at least one single nucleotide polymorphic site as a diagnostic marker.

14. A method for diagnosing a disease in a subject, comprising identifying a single nucleotide at one or more single nucleotide polymorphic sites according to the method of any one of claims 1 to 9 in a nucleic acid sample of the subject, and determining whether the identified one or more nucleotides correspond to a single base polymorphism associated with said disease.

15. A kit for identifying single nucleotides at specific positions in at least one target nucleic acid, comprising a first DNA microarray and a second DNA microarray as defined in any one of claims 1 to 6.
